**Europäisches Patentamt**

**European Patent Office**

**Office européen des brevets**

(11) Veröffentlichungsnummer : **0 332 884 B1**

# (12) EUROPÄISCHE PATENTSCHRIFT

(45) Veröffentlichungstag der Patentschrift :
**29.04.92 Patentblatt 92/18**

(51) Int. Cl.$^5$ : **A61M 25/00**

(21) Anmeldenummer : **89102861.5**

(22) Anmeldetag : **18.02.89**

(54) **Ventil für Venenverweilkanülen oder Kathetereinführvorrichtungen.**

(30) Priorität : **18.03.88 DE 3809127**

(43) Veröffentlichungstag der Anmeldung :
**20.09.89 Patentblatt 89/38**

(45) Bekanntmachung des Hinweises auf die
Patenterteilung :
**29.04.92 Patentblatt 92/18**

(84) Benannte Vertragsstaaten :
**AT BE CH DE ES FR GB GR IT LI LU NL SE**

(56) Entgegenhaltungen :
**DE-A- 2 817 102**
**US-A- 3 788 598**
**US-A- 4 334 551**

(73) Patentinhaber : **B. Braun Melsungen AG**
**Carl-Braun Strasse**
**W-3508 Melsungen (DE)**

(72) Erfinder : **Haindl, Hans, Dr.**
**Forstgarten 22**
**W-3508 Melsungen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

## Beschreibung

Die Erfindung betrifft ein Ventil nach dem Oberbegriff des Patentanspruchs 1, wie es beispielsweise aus der DE-A-28 17 102 bekannt ist.

Zum Einbringen eines Venenkatheters in ein Blutgefäß wird das Gefäß zunächst mit einer Venenverweilkanüle punktiert. Solche Venenverweilkanülen bestehen aus einer Stahlkanüle und einer darübergeschobenen Kunststoffkanüle. Sobald der Punktionserfolg eintritt, wird die Stahlkanüle aus der Kunststoffkanüle zurückgezogen. Durch die dann im Gefäß verbleibende Kunststoffkanüle kann der Venenkatheter eingeschoben werden. Es besteht auch die Möglichkeit, die Kunststoffkanüle als Kurzkatheter im Blutgefäß zu belassen.

Nachdem im Anschluß an eine Gefäßpunktion die Stahlkanüle aus der Kunststoffkanüle herausgezogen wurde, kann Blut aus der Kunststoffkanüle austreten. Im ungünstigsten Fall, wenn durch Hochlagerung des Oberkörpers oder durch extreme Austrocknung des Patienten der zentrale Venendruck negativ ist, kann auch Luft in den Körper des Patienten eintreten, mit der Gefahr des Entstehens einer Luftembolie.

Um die beiden Risiken "Luftembolie" und "Blutkontamination des Arztes" zu vermeiden, ist aus der eingangs aufgeführten DE-A-28 17 102 ein in das Anschlußstück einer Venenverweilkanüle integriertes Ventil bekannt, das nur durch mechanischen Druck in Richtung zum Patienten geöffnet werden kann. Als Ventilkörper dient eine geschlitzte Latex-Scheibe. Im Lieferzustand der Venenverweilkanüle ist die Stahlkanüle durch die Scheibe hindurchgeführt. Nach erfolgter Punktion und anschließendem Zurückziehen der Stahlkanüle schließt sich die Scheibe infolge ihrer Rückstellkraft. Anschließend kann ein Luer-Konnektor an das Anschlußstück angesetzt werden, der mit seinem konischen Rohransatz die Scheibe aufstößt, so daß diese für das Einführen eines Venenkatheters geöffnet ist. Bei diesem bekannten Ventil treten Probleme mit der Lagerfähigkeit auf, weil das Ventil mit hindurchgesteckter Stahlkanüle gelagert wird. Das Elastomermaterial der Scheibe verliert im Laufe der Zeit seine Rückstellfähigkeit, so daß die Gefahr besteht, daß das Ventil nach längerer Lagerung nicht mehr schließt. In der Praxis macht man daher die Stahlkanüle in dem Bereich, in dem sie von der elastischen Scheibe umschlossen ist, dünner als im übrigen Verlauf. Dadurch wird das Ventil während der Lagerung nur geringfügig aufgespreizt. Es besteht allerdings die Gefahr, daß sich an der Verengungsstelle der Stahlkanüle Gewebspartikel und Thromben des Patienten festsetzen, die dann diese Engstelle verstopfen.

Der Erfindung liegt die Aufgabe zugrunde, ein Ventil der im Oberbegriff des Patentanspruchs 1 angegebenen Art so weiterzubilden, das auch nach langer Öffnungszeit auf einer ungeschwächten Stahlkanüle nach dem Entfernen dieser Stahlkanüle noch sicher schließt.

Die Lösung dieser Aufgabe erfolgt bei einem Ventil der eingangs genannten Art erfindungsgemäß mit dem im kennzeichnenden Teil des Patentanspruchs 1 angegebenen Merkmal.

Nach der Erfindung ist zusätzlich zu der elastomeren Ventilkörper eine separate Feder, vorzugsweise aus Stahl, vorgesehen, die den Ventilkörper in die Schließposition spannt. Im Gegensatz zu Elastomeren verlieren Federstahlwerkstoffe ihre elastischen Eigenschaften auch bei langer Lagerzeit nicht oder in erheblich geringerem Maße. Durch die zusätzliche Feder wird der Ventilkörper auch nach längerer Lagerzeit sicher in die Schließposition gedrückt, nachdem die Stahlkanüle aus dem Ventil herausgezogen wurde.

Anspruch 2 bezieht sich auf eine besonders einfache, wenig Platz beanspruchende und kostengünstige Ausbildung der Feder, die als axial federnde Tellerfeder ausgebildet ist.

Bei der Ausführungsform nach Anspruch 3 wirkt zum Öffnen des Ventilkörpers der Rohransatz einer externen Einrichtung unmittelbar auf den Ringansatz der Scheibe ein, der zur patientenfernen Seite hin absteht.

Nach Anspruch 5 ist in dem Kanal ein längsverschiebbarer Schiebekörper vorgesehen, der ebenfalls von einer separaten Feder (aus Federstahl) vorgespannt ist, und zwar in die Rückzugsposition, in der er außer Eingriff mit der Scheibe ist.

Zum Geschlossenhalten des Ventilkörpers kann auch eine andere Metallfeder verwendet werden, beispielsweise eine Schraubenfeder, die sich am Gehäuse abstützt und mit axialer Komponente gegen die geöffnete Scheibe drückt.

Im folgenden werden unter Bezugnahme auf die Zeichnungen zwei Ausführungsbeispiele der Erfindung näher erläutert.

Es zeigen:

Fig. 1 einen Längsschnitt des Ventils im Ruhezustand,

Fig. 2 einen Längsschnitt des Ventils im Lieferzustand, mit eingeschobener Stahlkanüle,

Fig. 3 den Ventilzustand nach dem Herausziehen der Stahlkanüle und während des Einführens eines Venenkatheters,

Fig. 4 eine Ansicht der Elastomerscheibe

Fig. 5 einen Längsschnitt durch eine zweite Ausführungsform des Ventils mit am Gehäuse abgestützter Schraubenfeder,

Fig. 6 eine Ausführungsform ähnlich derjenigen von Fig. 5, jedoch mit einer zusammen mit dem Ventilkör-

2

per eingespannten Tellerfeder,

Fig. 7 eine Ansicht der Tellerfeder und

Fig. 8 eine Teil-Darstellung des Ventils von Fig. 6 im Öffnungszustand.

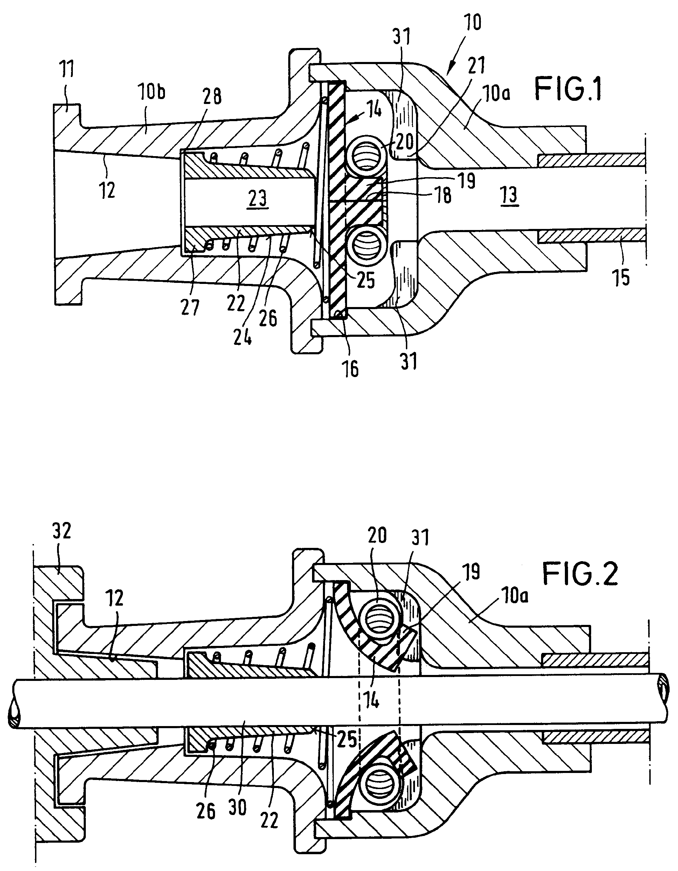

Das in den Fign. 1 bis 4 dargestellte Ventil weist ein zweiteiliges langgestrecktes Ventilgehäuse 10 auf, mit einem patientenseitigen Gehäuseteil 10a und einem patientenfernen Gehäuseteil 10b, welche fest und abdichtend miteinander verbunden sind. Am Gehäuseteil 10b sind Anschlußelemente 11 für einen Luer-Lok-Konnektor vorgesehen, wobei die Einführöffnung 12 als Innenkonus ausgebildet ist, in den ein Rohransatz einführbar ist.

Durch das Gehäuse 10 erstreckt sich in Längsrichtung der Kanal 13, der durch den elastomeren Ventilkörper 14 in Längsrichtung unterteilt ist. Von dem patientenseitigen Ende des Gehäuses 10 steht die Kunststoffkanüle 15 ab, die entweder als Einführhilfe für einen Katheter oder unmittelbar als Kurzkatheter verwendbar ist.

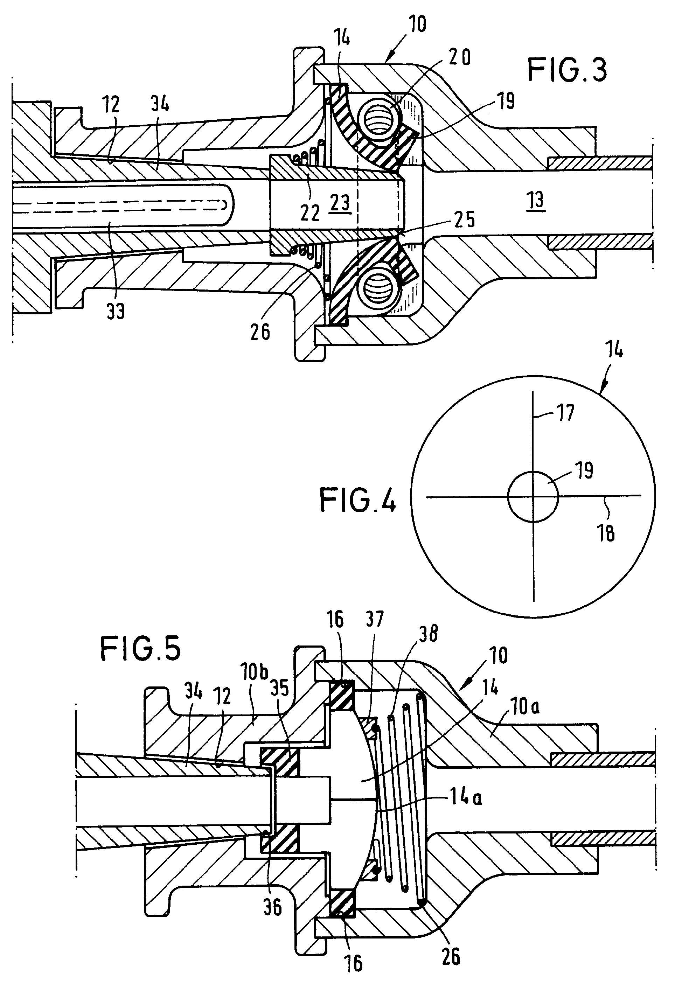

Der scheibenförmige Ventilkörper 14 ist an seinem Rand in einer umlaufenden Nut 16 zwischen den beiden Gehäuseteilen 10a und 10b fest eingespannt. Wie aus Fig. 4 ersichtlich ist, ist der Ventilkörper 14 kreuzförmig oder Y-förmig geschlitzt, wobei die Schlitze mit 17 und 18 bezeichnet sind. Im Mittelbereich des Ventilkörpers 14 ist ein zum patientenseitigen Ende abstehen der Ansatz 19 bzw. eine Warze vorgesehen, in den bzw. die hinein die Schlitze 17 und 18 sich ebenfalls erstrecken. Dieser Ansatz 19 ist von einer Ringfeder 20 nach Art eines Spannringes umgeben, der die Scheibe in den Schließzustand spannt, in welchem die Schlitze 17 und 18 geschlossen sind. Hinter dem Ventilkörper 14 ist ein Ausweichraum 21 im Gehäuseteil 10a vorgesehen, in den die Segmente der Scheibe und die Ringfeder 20 im Öffnungszustand radial und axial ausweichen können.

In dem Kanal 13 ist vor dem Ventilkörper 14 ein Schiebekörper 22 angeordnet, der einen längslaufenden Durchlaß 23 und eine konische Außenfläche 24 aufweist. Das konische vordere Ende 25 des Schiebekörpers 22 ist auf den Mittelbereich des Ventilkörpers 14 gerichtet. Der Schiebekörper 22 wird durch eine metallische Schraubenfeder 26, die gegen den Flansch 27 des Schiebekörpers drückt, von dem Ventilkörper 14 fortgedrückt. Die Schraubenfeder 26 hat konischen bzw. glockenförmigen Verlauf und ist mit ihrer Hüllfläche der Innenfläche des Gehäuseteils 10b angepaßt. Das größere Schraubenende ist zwischen Ventilkörper 14 und Gehäuseteil 10b festgeklemmt. Zur Begrenzung der Ruhestellung des Schiebekörpers 22 dient ein Anschlag 28 im Gehäuseteil 10b.

Fig. 1 zeigt das Ventil in seiner Ruhestellung, in der der Ventilkörper 14 geschlossen ist und von der Ringfeder 20 im Schließzustand gehalten wird. Der Schiebekörper 22 befindet sich in seiner Rückzugsposition.

In Fig. 2 ist der Zustand dargestellt, daß eine Stahlkanüle 30 durch das Ventil hindurchgeschoben ist. Die Stahlkanüle 30 verläuft durch den Schiebekörper 22 hin durch, ohne diesen mitzunehmen. Sie stößt den Ventilkörper 14 auf, so daß deren Segmente zum patientenseitigen Ende hin ausweichen und die Ringfeder 20 aufgeweitet wird. An dem Gehäuseteil 10a sind Rippen 31 vorgesehen, die in die Lücken zwischen den aufgespreizten Segmenten des Ventilkörpers 14 hineinragen und verhindern, daß die Ringfeder 20 axial von dem Ansatz 19 abgleitet. Die Rippen 31 stützen die Ringfeder 20 sowohl im entspannten Zustand als auch im gespannten Zustand vom patientenseitigen Ende her ab. In Fig. 2 ist der Lagerzustand des Ventils dargestellt, in dem die Stahlkanüle 30 im Kanal 13 sitzt, während ein Konnektor 32, durch den die Stahlkanüle hindurchgeht, in der Einführöffnung 12 befestigt ist. In diesem Zustand erfolgt die Punktion des Blutgefäßes mit der Stahlkanüle, welche von der Kunststoffkanüle 15 umgeben ist. Wird anschließend die Stahlkanüle 30 zurückgezogen, dann schließt sich der Ventilkörper 14 unter der Wirkung der Ringfeder 20, so daß weder Blut aus dem Gehäuse 10 austreten noch Luft in die Kunststoffkanüle 15 eindringen kann.

Fig. 3 zeigt das Ventil beim Einführen eines Katheters 33 durch das geöffnete Ventil und die Kunststoffkanüle 15 hindurch in den Körper. Das Öffnen des Ventils erfolgt dadurch, daß der kegelförmige Rohransatz 34 der Einführvorrichtung, aus der heraus der Katheter 33 vorgeschoben wird, in die Einführöffnung 12 eingeschoben wird. Der Rohransatz 34 ist so lang, daß er gegen das rückwärtige Ende des Schiebekörpers 22 drückt und diesen vorschiebt, wodurch unter axialer Zusammendrückung der Feder 26 das vordere Ende 25 des Schiebekörpers den Ventilkörper 14 öffnet. Der Innendurchmesser des Rohransatzes 34 entspricht etwa dem Durchmesser des Kanals 23 des Schiebekörpers 22. Der Katheter 33 kann somit innerhalb des Schiebekörpers 22 durch den geöffneten Ventilkörper 14 hindurchgeschoben werden. Wird anschließend der Rohransatz 34 zurückgezogen, dann umschließen die Segmente des Ventilkörpers 14 den Katheter 33, weil der Schiebekörper 22 in seine Rückzugsposition zurückkehrt.

Fig. 5 zeigt eine Ausführungsform, bei der ein Schiebekörper nicht vorhanden ist, unmittelbar vor dem Einführen eines Katheters. Der Rohransatz 34 stößt unmittelbar gegen einen den Ventilkörper 14 einstückig angeformten Ringansatz 35, der vom Ventilkörper 14 zum rückwärtigen Ende hin absteht. Im Ringansatz 35 ist eine entsprechende Nut 36 zur Aufnahme des vorderen Endes des Rohransatzes 34 ausgebildet. Beim Ansetzen des Rohransatzes 34 drückt dieser unmittelbar gegen den Ringansatz 35 des Ventilkörpers 14, so daß ein zwischengeschalteter Schiebekörper nicht erforderlich ist. Der Ventilkörper 14 hat an seinem stromabwärts gerichteten Ende eine ballige Außenfläche 14a, gegen die ein Adapterring 37 gesetzt ist. Gegen diesen Adapterring 37 drückt eine kegelstumpfförmige Schraubenfeder 38, die am Gehäuse 10 abgestützt ist und die durch die Schlitze voneinander getrennten Segmente des Ventilkörpers in den Schließzustand drückt. Der Adapterring 37 greift am Umfang der Segmente des Ventilkörpers an. Beim Aufstoßen des Ventilkörpers 14 gleiten die Segmente dieses Ventilkörpers an dem Adapterring 37 und im Ventilkörper entsteht eine Öffnung, die sich durch den Adapterring 37 erstreckt.

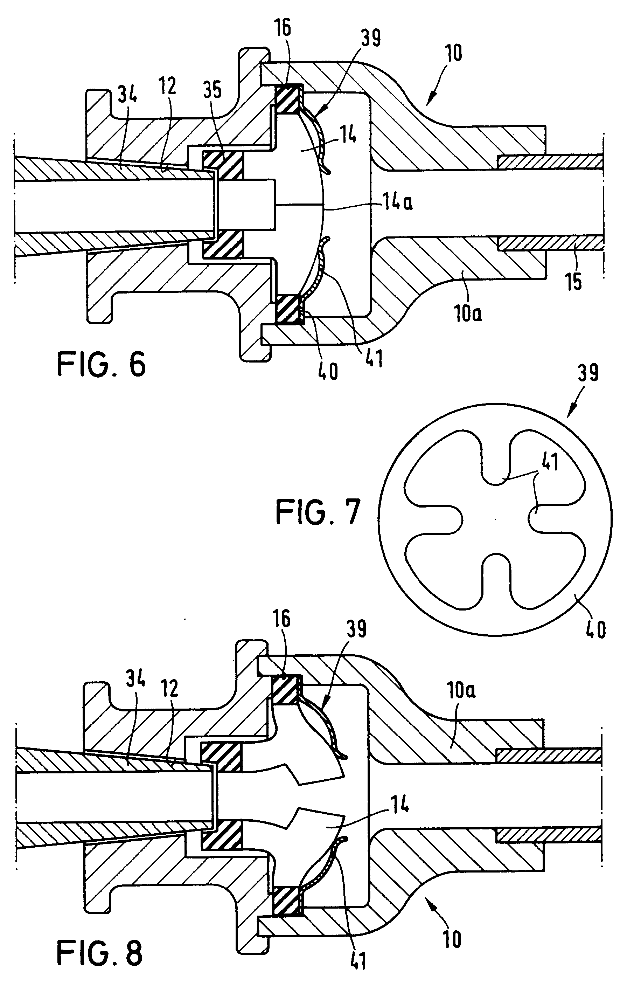

Bei dem Ausführungsbeispiel der Fign. 6 bis 8 ist der Ventilkörper 14 in gleicher Weise ausgebildet, wie bei dem Ausführungsbeispiel nach Fig. 5, jedoch ist die Feder eine Blattfeder 39 mit einem scheibenförmigen Ring 40, von dem federnde Arme 41 radial nach innen weisen. Die federnden Arme 41 drücken gemäß Fig. 6 gegen die ballige Außenseite 14a des Ventilkörpers 14 und spannen den Ventilkörper in die Schließstellung.

Fig. 8 zeigt den Öffnungszustand, in dem der Ventilkörper durch den Rohransatz 34 aufgestoßen wird. Dabei drücken die Segmente des Ventilkörpers 14 die federnden Arme 41 der Tellerfeder 39 auseinander. Der Ring 40 der Tellerfeder ist zusammen mit dem Umfangsrand des Ventilkörpers 14 in der Nut 16 des Gehäuses 10 eingespannt.

Bei den beschriebenen Ausführungsbeispielen erfolgt das Öffnen des Ventilkörpers 14 jeweils durch axiales Vorschieben des Rohransatzes 34. Es besteht auch die Möglichkeit, am Rohransatz 34 eine Überwurfmutter anzubringen, die mit Gewindeteilen des Gehäuses 10 in Eingriff steht, so daß die Öffnungsstellung des Ventils durch Drehen der Überwurfmutter verändert werden kann. Durch Drehen der Überwurfmutter in Gegenrichtung kann der Ventilkörper 14 wieder verschlossen werden. Um eine derartige Ventilanordnung abzudichten, kann es erforderlich sein, einen Dichtring zwischen der Einführöffnung 12 und dem Rohransatz 34 vorzusehen.

## Patentansprüche

1. Ventil für Venenverweilkanülen oder Kathetereinführvorrichtungen, mit
einem Gehäuse (10), welches einen längslaufenden Kanal (13) aufweist,
einen den Kanal (13) unterteilenden geschlitzten elastomeren Ventilkörper (14), welcher zur Einnahme der Öffnungsposition zum patientenseitigen Ende hin aufdrückbar ist,
und einer an dem patientenfernen Ende des Kanals (13) vorgesehenen Einführöffnung (12) zum Einführen eines den Ventilkörper (14) aufdrückenden Rohransatzes (34),
**dadurch gekennzeichnet,**
daß eine den Ventilkörper (14) in seine Schließposition vorspannende Feder (20,38,39) vorgesehen ist.
2. Ventil nach Anspruch 1, dadurch gekennzeichnet, daß die Feder (39) eine an ihrem Umfang eingespannte und mit nach innen gerichteten Armen (41) gegen den Ventilkörper drückende Tellerfeder ist.
3. Ventil nach Anspruch 1 oder 2, dadurch gekennzeichnet, daß der Ventilkörper (14) am patientenfernen Ende einen Ringansatz (35) aufweist, gegen den der Rohransatz (34) unmittelbar ansetzbar ist.
4. Ventil nach Anspruch 1, dadurch gekennzeichnet, daß die Feder (20) eine Ringfeder ist, welche die Segmente eines von der Ventilkörpermitte axial ab stehenden, ebenfalls geschlitzten Ansatzes (19) radial zusammenpreßt.
5. Ventil nach einem der Ansprüche 1 bis 4, dadurch gekennzeichnet, daß in dem patientenfernen Teil des Kanals (13) ein Schiebekörper (22) längsverschiebbar angeordnet ist, dessen eines Ende (25) gegen den Mittelbereich des Ventilkörpers (14) drückbar ist, und daß der Schiebekörper (22) von einer weiteren Feder (26) in seine Rückzugsposition vorgespannt ist.
6. Ventil nach einem der Ansprüche 1 bis 5, dadurch gekennzeichnet, daß das Gehäuse (10) einen Ausweichraum für die auseinandergespreizten Segmente des Ventilkörpers (14) aufweist, und daß in den Ausweichraum Rippen (31) vorstehen, die zwischen die Segmente des Ventilkörpers (14) ragen und ein Abgleiten der Feder (20) von dem Ansatz (19) der Scheibe (14) verhindern.
7. Ventil nach Anspruch 1, dadurch gekennzeichnet, daß die Feder (38) eine am Gehäuse (10) abgestützte Schraubenfeder ist, die über ein Adapterelement (37) gegen den Ventilkörper (14) drückt.

## Claims

1. A valve for dwell-in venous cannulas or catheter insertion means, comprising
a housing (10) having a longitudinally extending channel (13),
a slotted elastomeric valve body (14), dividing the channel (13), which valve body can be pushed open toward the patient to take an opened position,

and an insertion means (12) provided at the end of the channel (13) remote from the patient, which insertion means (12) is provided for inserting a pipe stud (34) that pushes open the valve body (14),

**characterised in that**

a spring (20,38,39) is provided that biasses the valve body (14) to its closed position.

2. The valve according to Claim 1, characterised in that the spring (39) is a disc spring chucked at its perimeter and pressing against the valve body with inward pointing arms (41).

3. The valve according to Claim 1 or 2, characterised in that the valve body (14) is provided with an annular collar (35) at the end averted from the patient, against which a pipe stud (34) is directly applicable.

4. The valve according to Claim 1, characterised in that the spring (20) is a conicla spring, which radially compresses the segments of a stud (19) that axially extends from the centre of the valve body, said stud (19) being also slotted.

5. The valve according to one of Claims 1 to 4, characterised in that the part of the channel (13) far from the patient is provided with a longitudinally displaceable sliding member (22), one end (25) of which can be pressed against the central part of the valve body (14) and that the sliding member (22) is biassed to its withdrawal position by a further spring (26).

6. The valve according to one of Claims 1 to 5, characterised in that the housing (10) is provided with a evasion space for the spread segments of the valve body (14) and that ribs (31) protrude into said evasion space, which ribs (31) extend between the segments of the valve body (14) and prevent a slipping of the spring (20) off the stud (19) of the disc (14).

7. The valve according to Claim 1, characterised in that the spring (38) is a coil spring supported on the housing (10), pressing against the valve body (14) via an adapter member (37).


**Revendications**

1. Soupape pour canules à demeure ou dispositifs d'introduction de cathéter, comportant

un carter (10) qui présente un canal (13) s'étendant dans la direction longitudinale,

un corps fendu formant soupape (14) en élastomère, qui subdivise le canal (13) et qui pour prendre la position d'ouverture, peut être poussé en direction de l'extrémité située côté patient pour s'ouvrir,

et une ouverture d'introduction (12) prévue au niveau de l'extrémité du canal (13), éloignée du patient, l'ouverture étant destinée à l'introduction d'un embout tubulaire (34) ouvrant le corps formant soupape (14) en le poussant,

caractérisée en ce qu'est

prévu un ressort (20, 38, 39) faisant subir au corps formant soupape (14) une précontrainte dans la direction de sa position de fermeture.

2. Soupape selon la revendication 1, caractérisée en ce que le ressort (39) est une rondelle élastique enserrée au niveau de sa périphérie et appuyant contre le corps formant soupape par l'intermédiaire de bras (41) dirigés vers l'intérieur.

3. Soupape selon la revendication 1 ou 2, caractérisée en ce que le corps formant soupape (14) comporte, au niveau de son extrémité éloignée du patient, une embase annulaire (35) contre laquelle peut être appliqué directement l'embout tubulaire (34).

4. Soupape selon la revendication 1, caractérisée en ce que le ressort (20) est un ressort annulaire comprimant radialement les segments d'une embase (19) qui est également fendue et qui fait saillie axialement au centre du corps formant soupape.

5. Soupape selon l'une des revendications 1 à 4, caractèrisée en ce que dans la partie du canal (13) éloignée du patient, est disposé de manière à pouvoir coulisser longitudinalement, un corps formant coulisseau (22) dont une extrémité (25) peut être poussée contre la zone centrale du corps formant soupape (14), et en ce que le corps formant coulisseau (22) est précontraint par un autre ressort (26), vers sa position de retrait.

6. Soupape selon l'une des revendications 1 à 5, caractérisée en ce que le carter (10) comporte une chambre de déport pour les segments du corps formant soupape (14) ayant été écartés les uns des autres, et en ce que dans la chambre de déport font saillie des nervures (31) qui s'engagent entre les segments du corps formant soupape (14) et qui empêchent le ressort (20) de s'échapper par glissement de l'embase (19) du disque (14).

7. Soupape selon la revendication 1, caractérisée en ce que le ressort (38) est un ressort hélicoïdal qui s'appuie sur le carter (10) et qui pousse sur le corps formant soupape (14), par l'intermédiaire d'un élément adaptateur (37).

FIG.1

FIG.2

FIG.3

FIG.4

FIG.5

FIG. 6

FIG. 7

FIG. 8